# EUROPEAN PATENT APPLICATION

(11) **EP 3 572 800 A1**
(43) Date of publication of application: **27.11.2019**
(21) Application number: 18742154.0
(22) Date of filing: 17.01.2018
(51) Int. Cl.: G01N 21/78, G01N 21/64, C09K 11/06

(54) **FLUORESCENT GEL FOR GLUCOSE DETECTION AND CONTINUOUS GLUCOSE MONITORING METHOD USING SAME**

(30) Priority: 19.01.2017 JP 2017007188; 20.10.2017 JP 2017204079
(71) Applicant: The University Of Tokyo, Tokyo 113-8654 (JP)
(72) Inventor: TAKEUCHI Shoji, Tokyo 113-8654 (JP); SAWAYAMA Jun, Tokyo 113-8654 (JP)
(74) Representative: Myint, Julie Marie
(86) International application number: PCT/JP2018/001239
(87) International publication number: WO 2018/135535

(57) **Abstract**

[Problem]

The present invention addresses the problem of providing: a novel fluorescent gel for glucose monitoring, whereby a concentration of glucose in a living body can be continuously measured and fibrosis of the gel can be suppressed even when the gel is implanted for a long period of time; and continuous glucose monitoring using the fluorescent gel.

[Solution]

A fluorescent gel for glucose monitoring, having a three-dimensional mesh structure in which a plurality of four-armed polymers having a polyethylene glycol skeleton are crosslinked at terminal ends thereof, wherein a fluorescent dye unit exhibiting a fluorescence response by bonding with glucose is immobilized in the gel structure by covalent bonding.

## Description

### TECHNICAL FIELD

The present invention relates to a novel fluorescent gel for glucose monitoring, whereby a glucose concentration in a living body can be continuously measured and fibrosis of the gel can be suppressed even when the gel is implanted for a long period of time, and to continuous glucose monitoring using the fluorescent gel.

### BACKGROUND ART

Control of blood sugar levels to a normal blood sugar region is important in the treatment of diabetes, for which the number of patients is rapidly increasing all over the world. There is therefore a need to develop a sensor whereby high precision of monitoring can be maintained over a long time period and a glucose concentration in a living body can be continuously measured and monitored.

However, enzyme-electrode-type measurement is currently a mainstream method for such glucose monitoring, but measurement by this method causes the enzyme to be consumed, and enzyme-electrode-type measurement cannot be used for long-term continuous measurement. Frequent blood sugar measurement by needle sampling is also necessary to maintain the desired precision of monitoring, which places a significant burden on a patient.

Meanwhile, methods for measuring a glucose response through use of a fluorescent dye in a gel are being researched (Non-patent Reference 1, etc.). Because the gel being used is primarily a polyacrylamide gel or other acrylic-based gel, when the gel is implanted in a living body, proteins and the like adhere to the gel, fibrosis or inflammation occurs as a result of xenobiotic reaction, and the gel cannot withstand prolonged use. Although introduction of functionality into a side chain of polyacrylamide or the like is being investigated, fibrosis and the like has not yet been adequately suppressed, and fluorescence intensity cannot follow fluctuation of blood glucose concentration. Furthermore, an acrylic-based gel is synthesized using radical polymerization, and production thereof is therefore subject to numerous limitations due to the use of anaerobic polymerization conditions, and the gel also has the problem of inadequate strength.

### PRIOR ART REFERENCES

### PATENT REFERENCES

Non-patent Reference 1: Shibata et al., Proc. Nat. Acad. Sci. USA, 107, pp. 17894-17898, 2010.

### SUMMARY OF THE INVENTION

### PROBLEMS TO BE SOLVED BY THE INVENTION

Therefore, an object of the present invention is to provide a novel fluorescent gel for glucose monitoring, whereby a glucose concentration in a living body can be continuously measured and fibrosis of the gel can be suppressed even when the gel is implanted for a long time period, and to provide continuous glucose monitoring using the fluorescent gel.

### MEANS USED TO SOLVE THE ABOVE-MENTIONED PROBLEMS

As a result of concentrated investigation aimed at overcoming the foregoing problems, the inventors discovered that by immobilizing a glucose-responsive fluorescent dye in a gel having a uniform network structure formed by a four-armed polyethylene glycol polymer (Tetra-PEG), it is possible to provide a novel fluorescent gel for glucose monitoring, whereby, adhesion of proteins and the like can be suppressed and fibrosis as a xenobiotic reaction can be reduced, and a glucose concentration in a living body can be continuously monitored over a long time period as a fluorescence emission response. The inventors also discovered that by immobilizing an enzyme or other antioxidant in the fluorescent gel, degradation of the fluorescent gel can be suppressed when the fluorescent gel is implanted in a living body, and characteristics of the abovementioned continuous monitoring can be further enhanced. The present invention was completed on the basis of these new findings.

Specifically, according to an aspect of the present invention, there are provided:
<1> a fluorescent gel for glucose monitoring, having a three-dimensional mesh structure in which a plurality of four-armed polymers having a polyethylene glycol skeleton are crosslinked at terminal ends thereof, wherein a fluorescent dye unit for exhibiting a fluorescence response by bonding with glucose is immobilized in the gel structure by covalent bonding;
<2> the fluorescent gel for glucose monitoring according to <1>, wherein the four-armed polymers comprise a first polymer having four nucleophilic functional groups at the terminal ends thereof, and a second polymer having four electrophilic functional groups at the terminal ends thereof;
<3> the fluorescent gel for glucose monitoring according to <2>, wherein the nucleophilic functional groups are selected from the group consisting of hydroxyl groups, amino groups, and thiol groups; and the electrophilic functional groups are selected from the group consisting of carboxyl groups, N-hydroxysuccinimidyl (NHS) groups, sulfosuccinimidyl groups, maleimidyl groups, phthalimidyl groups, and nitrophenyl groups;
<4> the fluorescent gel for glucose monitoring according to <2>, wherein the first polymer has a structure represented by formula (I) 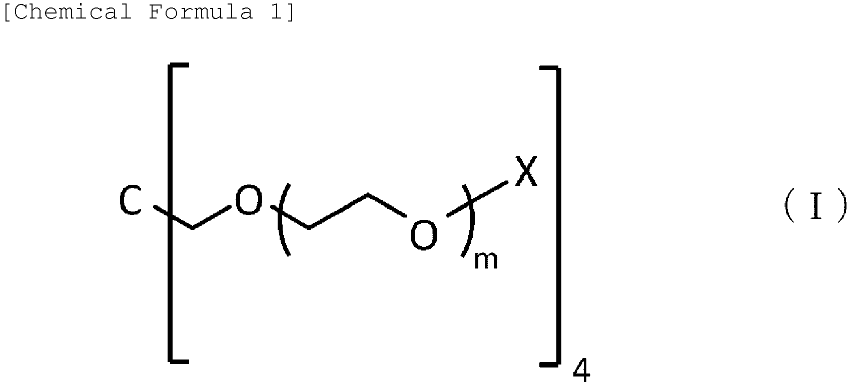
   (In formula (I), each m is the same or different and is 10 to 300, each X is R¹-NH₂ or R¹-SH, and R¹ is a C₁-C₇ alkylene group); and
   the second polymer has a structure represented by formula (II) 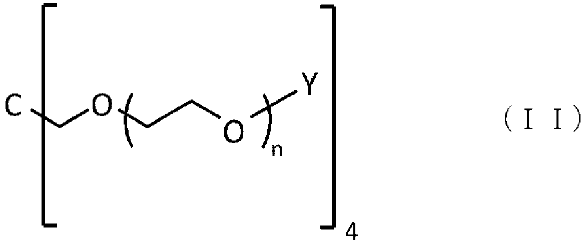
   (In formula (II), each n is the same or different and is 10 to 300, and each Y is -CO-R²-COO-NHS or an R²-maleimidyl group, R² being a C₁-C₇ alkylene group.);
<5> the fluorescent gel for glucose monitoring according to any one of <1> through <4>, wherein the fluorescent dye unit has a fluorophore and a quencher; and the fluorophore is quenched by the quencher when glucose is not present, but by of bonding of the quencher with glucose, the fluorophore emits light and thereby exhibits a fluorescence response;
<6> the fluorescent gel for glucose monitoring according to <5>, wherein the fluorophore has anthracene and the quencher has arylboronic acid;
<7> the fluorescent gel for glucose monitoring according to any one of <1> through <6>, wherein the fluorescent dye unit has one or a plurality of amino groups at a terminal end thereof, and is immobilized in the gel structure by covalent bonding of the amino group and the four-armed polymers;
<8> the fluorescent gel for glucose monitoring according to any one of <1> through <7>, wherein the fluorescent dye unit is represented by formula (III) or formula (IV) below: 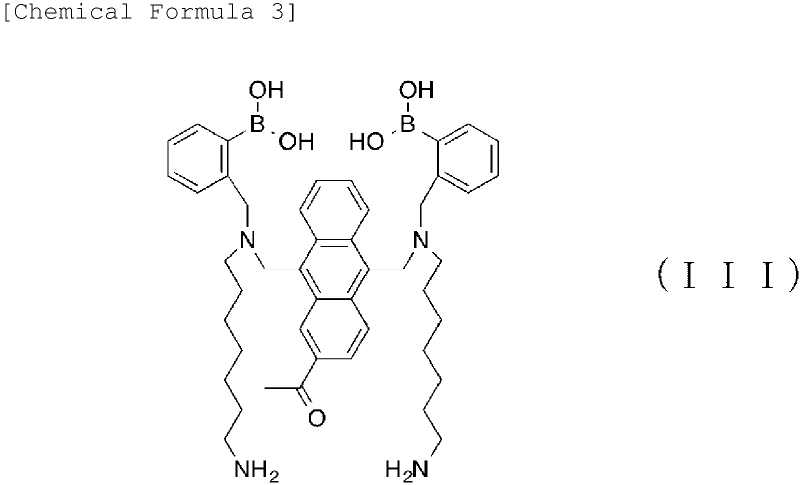 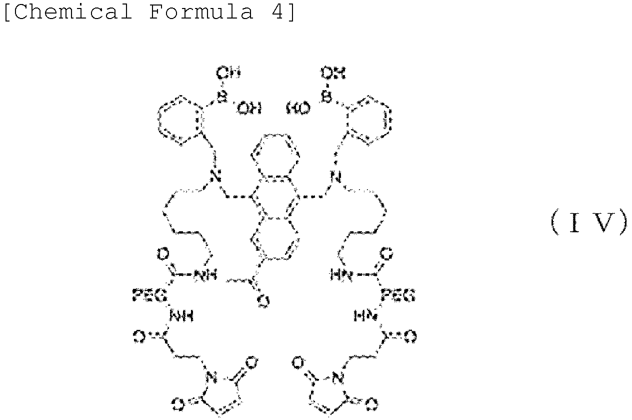 (In formula (IV), "PEG" represents a polyethylene glycol chain.);
<9> the fluorescent gel for glucose monitoring according to any one of <1> through <8>, further including an antioxidant;
<10> the fluorescent gel for glucose monitoring according to any one of <1> through <9>, wherein the antioxidant is immobilized in the gel structure by covalent bonding;
<11> the fluorescent gel for glucose monitoring according to <9> or <10>, wherein the antioxidant is an enzyme; and
<12> the fluorescent gel for glucose monitoring according to <11>, wherein the enzyme is catalase, SOD, peroxiredoxin, metallothionein, glutathione peroxidase, glutathione, thioredoxin, or glucose-6-phosphate dehydrogenase.

According to another aspect of the present invention, there are provided:
<13> an implantable glucose monitoring sensor comprising the fluorescent gel for glucose monitoring according to any one of <1> through <12>;
<14> a glucose monitoring method comprising a step for detecting, as a fluorescence response, a presence of glucose in a sample by using the fluorescent gel for glucose monitoring according to any one of <1> through <12>; and
<15> a method for continuous monitoring of glucose, characterized in that the presence of glucose in a sample is continuously monitored by the glucose monitoring method according to <14>.

### ADVANTAGES OF THE INVENTION

The present invention exhibits the effect that fibrosis of the gel can be suppressed even when the gel is implanted for use in a living body, and continuous glucose monitoring is possible in which high precision of monitoring is maintained over a long time period. The present invention can therefore be used as an *in vivo* vital sign measurement sensor for performing highly precise continuous monitoring of a blood sugar level, which fluctuates moment by moment in a diabetes patient, the present invention thereby affording enhanced QOL, prevention of disease complication, and other effects.

By immobilizing an enzyme or other antioxidant in the fluorescent gel in addition to the glucose-responsive fluorescent dye unit, degradation of the fluorescent gel due to fibrosis and the like when the fluorescent gel is implanted in a living body can be further suppressed, and durability and characteristics of the abovementioned continuous monitoring can be further enhanced.

Furthermore, in addition to being biocompatible, the fluorescent gel for glucose monitoring according to the present invention has the advantages of having high mechanical strength and of being produced by a simple method, and therefore also has extremely high industrial utility value.

### BRIEF DESCRIPTION OF THE DRAWINGS

[FIG. 1] Schematic diagrams (a through c) illustrating steps for producing a fluorescent gel for glucose monitoring according to the present invention, and an image (d) of the fluorescent gel for glucose monitoring according to the present invention.
[FIG. 2] A graph indicating results of *in vitro* evaluation of glucose responsiveness of the fluorescent gel (TAPEG-TNPEG) for glucose monitoring according to the present invention.
[FIG. 3] A graph indicating results of *in vitro* evaluation of glucose responsiveness of the fluorescent gel (TTPEG-TMPEG) for glucose monitoring according to the present invention.
[FIG. 4] An image showing a configuration of an implantable device including the fluorescent gel for glucose monitoring according to the present invention.
[FIG. 5] A graph indicating results of *in vivo* evaluation of glucose responsiveness of the fluorescent gel (TAPEG-TNPEG) for glucose monitoring according to the present invention.
[FIG. 6] Graphs indicating results (left diagram) of a comparative example using a polyacrylamide gel and results (right diagram) of using the fluorescent gel for glucose monitoring according to the present invention in which an enzyme is immobilized, for an *in vivo* evaluation of glucose responsiveness.
[FIG. 7] A graph indicating results of evaluation of glucose responsiveness 14 days after implantation, for the fluorescent gel for glucose monitoring according to the present invention in which an enzyme is immobilized.
[FIG. 8] A graph indicating results of evaluation of glucose responsiveness 28 days after implantation, for the fluorescent gel for glucose monitoring according to the present invention in which an enzyme is immobilized.
[FIG. 9] A graph indicating glucose permeation rate 28 days after implantation, for the fluorescent gel for glucose monitoring according to the present invention in which an enzyme is immobilized.
[FIG. 10] A graph indicating results of evaluation of glucose responsiveness in a hypoglycemic region, using the fluorescent gel for glucose monitoring according to the present invention in which an enzyme is immobilized.
[FIG. 11] A graph indicating results of performing glucose monitoring in a diabetic mouse for one week.

### BEST MODE FOR CARRYING OUT THE INVENTION

Embodiments of the present invention are described below. The scope of the present invention is not limited to the above described embodiments, and modifications other than those of the examples described below may be made, as appropriate, insofar as the intent of the present invention is not compromised.

A fluorescent gel for glucose monitoring according to the present invention is characterized in that:
1) the fluorescent gel has a structure in which a gel is formed by a three-dimensional network structure in which a plurality of four-armed hydrophilic polymers having a polyethylene glycol skeleton are crosslinked at terminal ends thereof, and;
2) a fluorescent dye unit (glucose-responsive fluorescent dye unit) capable of exhibiting a fluorescence response by bonding with glucose is immobilized by covalent bonding in the gel structure formed by the four-armed polymers.

### 1. Four-armed polymer component having a polyethylene glycol skeleton

In the fluorescent gel for glucose monitoring according to the present invention, polymer components constituting the gel structure each have a polyethylene glycol skeleton having an ether chain branched into four branches. The polymers are crosslinked with each other at the terminal ends thereof, whereby a uniform three-dimensional mesh structure is formed, and the polymers are gelated. The gel includes water, and thus forms a hydrogel. The term "hydrogel" herein refers to a gelled substance which includes a hydrophilic macromolecule including a large quantity of water, and the term "gel" refers in general to a highly viscous dispersion from which fluidity has been lost.

In the fluorescent gel of the present invention, terminal ends of the four polyethylene glycol branches of each of the plurality of polymer components are crosslinked with each other to form a uniform mesh-structured network. A gel comprising such a four-armed polyethylene glycol skeleton is commonly known as Tetra-PEG gel, and a mesh-structured network is constructed therein by an AB-type cross-end coupling reaction between two types of four-armed macromolecules having an active ester structure or other electrophilic functional group and an amino group or other nucleophilic functional group at respective terminal ends thereof. Tetra-PEG gel can easily be prepared on-site by simple two-liquid mixing of macromolecular solutions, and also has excellent biocompatibility due to having polyethylene glycol as the main component thereof.

Consequently, the four-armed polymers constituting the fluorescent gel for glucose monitoring according to the present invention comprise a first polymer having nucleophilic functional groups at the terminal ends thereof, and a second polymer having electrophilic functional groups at the terminal ends thereof. Terminal ends of the first polymer and terminal ends of the second polymer undergo a crosslinking reaction (AB-type cross-end coupling reaction) by covalent bonding to form a mesh-structured network, forming a gel. For example, when the terminal ends of the first polymer are amino groups and the terminal ends of the second polymer are ester structures including N-hydroxysuccinimidyl (NHS), the terminal ends of the polymers crosslink by amide bonding, and a gel is formed. Preferably, the first polymer has four nucleophilic functional groups at the terminal ends of the four branches thereof, and the second polymer has four electrophilic functional groups at the terminal ends of the four branches thereof.

The nucleophilic functional groups present at the terminal ends of the first polymer are preferably hydroxyl groups, amino groups, or thiol groups, and more preferably amino groups or thiol groups. However, insofar as crosslinking is obtained whereby a gel can be formed, nucleophilic functional groups other than the above examples can be used as the nucleophilic functional groups, and a person skilled in the art could, as appropriate, use a publicly known nucleophilic functional group. The nucleophilic functional groups may each be the same or different, but are preferably the same. Having the functional groups be the same provides uniform reactivity with the nucleophilic functional group to be reacted with in crosslinking, and facilitates obtaining a high-strength gel having a uniform three-dimensional structure.

The electrophilic functional groups present at the terminal ends of the second polymer are preferably functional groups selected from the group consisting of carboxyl groups, N-hydroxysuccinimidyl (NHS) groups, sulfosuccinimidyl groups, maleimidyl groups, phthalimidyl groups, and nitrophenyl groups. The electrophilic functional groups are more preferably NHS groups or maleimidyl groups. However, other electrophilic active ester groups may be used, and a person skilled in the art could, as appropriate, use a publicly known active ester group. The functional groups may each be the same or different, but are preferably the same. Having the functional groups be the same provides uniform reactivity with the nucleophilic functional group to be reacted with in crosslinking, and facilitates obtaining a high-strength gel having a uniform three-dimensional structure.

The polymers can also be crosslinked using a so-called click reaction, instead of a crosslinking reaction by combination of the electrophilic functional groups and nucleophilic functional groups present at the terminal ends of the polymers. For example, the abovementioned polymers can be crosslinked with each other to form a gel using a click reaction in which an azide group and an alkyne group are combined. Such a click reaction is a 1,3-dipolar cycloaddition, and both the azide group and the alkyne group therefore act in nucleophilic and electrophilic fashion.

In an embodiment of the present invention, the first polymer used in the fluorescent gel for glucose monitoring is a four-armed polymer having a polyethylene glycol skeleton represented by general formula (I) below.

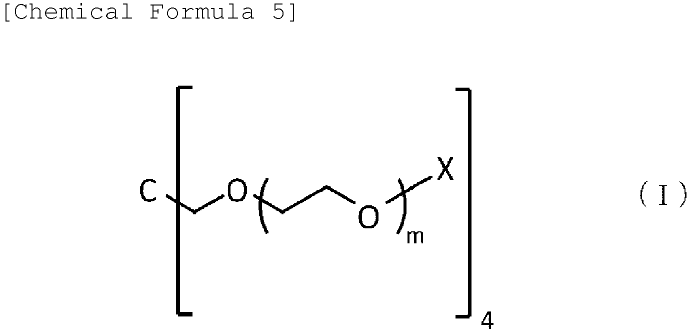

In formula (I), each m is the same or different and is 10 to 300, preferably 25 to 75, each X is R¹-NH₂ or R¹-SH, and R¹ is a C₁-C₇ alkylene group.

Here, the term "C₁-C₇ alkylene group" means an alkylene group having a carbon number of 1 to 7 which may have branching, and means a straight-chain C₁-C₇ alkylene group or a C₂-C₇ alkylene group (the carbon number including branches being 2 to 7) having one or more branches. Styrene, ethylene, propylene, and butylene groups are examples of C₁-C₇ alkylene groups. Examples of C₁-C₇ alkylene groups include -CH₂-,-(CH₂)₂-, -(CH₂)₃-, -CH(CH₃)-, -(CH₂)₃-, -(CH(CH₃))₂-, -(CH₂)₂-CH(CH₃)-, -(CH₂)₃-CH(CH₃)-, -(CH₂)₂-CH(C₂H₅)-, -(CH₂)₆-, -(CH₂)₂-C(C₂H₅)₂-, -(CH₂)₃C(CH₃)₂CH₂-, and the like. In the present specification, the alkylene group may have one or more of any substituent. An alkoxy group, a halogen atom (which may be a fluorine atom, a chlorine atom, a bromine atom, or an iodine atom), an amino group, a mono- or disubstituted amino group, a substituted silyl group, an acyl group, or an aryl group or the like can be cited as an example of the substituent, but these examples are not limiting. When the alkylene group has two or more substituents, the substituents may be the same or different.

In formula (I) above, each m may be the same or different, but the closer the m values are to each other, the more uniform the three-dimensional structure can be, and the higher is the strength thereof. Therefore, the m values are preferably the same in order to obtain a high-strength gel. The strength of the gel is reduced when the m values are too high, and a gel is not readily formed due to steric hindrance of compounds when the m values are too low. From the perspective of causing voids in the gel to have large enough space to take in glucose, m is 10 to 300, and preferably 25 to 75, as described above.

In an embodiment of the present invention, the second polymer used in the fluorescent gel for glucose monitoring is a four-armed polymer having a polyethylene glycol skeleton represented by general formula (II) below.

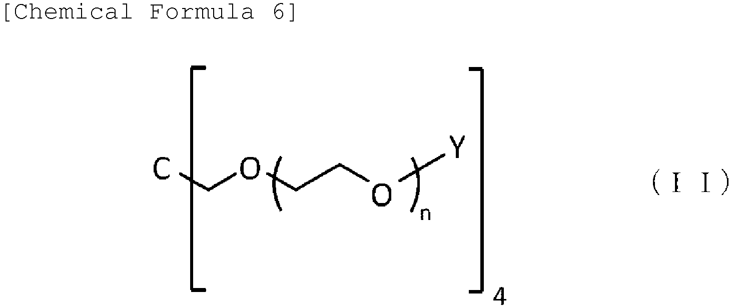

In formula (II), each n is the same or different and is 10 to 300, and each Y is -CO-R²-COO-NHS or an R²-maleimidyl group, R² being a C₁-C₇ alkylene group.

As in formula (I) above, each n may be the same or different, but the closer the n values are to each other, the more uniform the three-dimensional structure can be, and the higher is the strength thereof. Therefore, the n values are preferably the same in order to obtain a high-strength gel. The strength of the gel is reduced when the n values are too high, and a gel is not readily formed due to steric hindrance of compounds when the n values are too low. From the perspective of causing voids in the gel to have large enough space to take in glucose, n is 10 to 300, and preferably 25 to 75, as described above.

### 2. Fluorescent dye unit component

In the fluorescent gel for glucose monitoring according to the present invention, the gel includes a fluorescent dye unit for exhibiting a fluorescence response by bonding with glucose. The presence of glucose in a sample can thereby be detected as a fluorescence response.

The fluorescent dye unit preferably has a fluorophore and a quencher. The "fluorophore" is a part of the fluorescent dye unit that includes a molecule which fluoresces when excited by a specific wavelength. The "quencher" is a part including an electron acceptor whereby light emission from the fluorophore can be reduced by interaction with the fluorophore, and in which, by bonding with glucose, the quenching action thereof is eliminated and light emission by the fluorophore is resumed. The fluorophore is thereby quenched by the quencher when glucose is not present, but by bonding of the quencher with glucose, the fluorophore emits light, and the presence of glucose can thereby be detected as an ON-OFF fluorescence response.

Preferably, the fluorophore has anthracene and the quencher has arylboronic acid. This condition is not limiting insofar as a fluorescence response is exhibited by bonding with glucose, and a combination of a fluorophore and a quencher that is publicly known in the technical field in question can also be used in some cases.

The fluorescent dye unit is preferably immobilized by covalent bonding in the gel structure. Consequently, the fluorescent dye unit has one or a plurality of amino groups at a terminal end thereof, and is immobilized in the gel structure by covalent bonding of the amino group and the four-armed polymers.

The compound represented by formula (III) below is cited as a preferred example of the fluorescent dye unit used in the fluorescent gel for glucose monitoring according to the present invention.

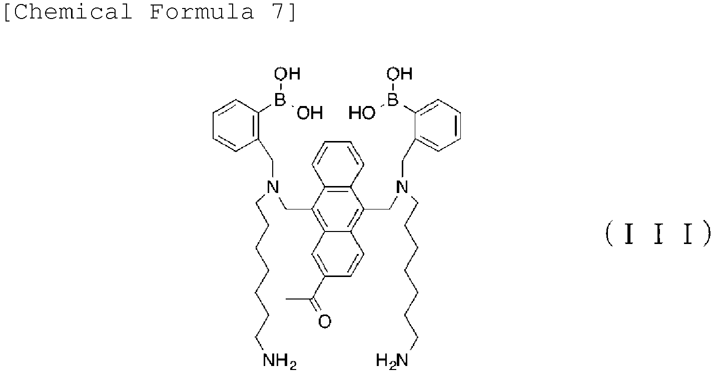

In the compound represented by formula (III), the anthracene in the center part is the fluorophore, and the two arylboronic acid groups on sides thereof are quenchers. As indicated in the balanced equation below, fluorescence of the anthracene is quenched by the arylboronic acid parts in the absence of glucose, but when bonding occurs between the arylboronic acid parts and glucose, movement of electrons to the anthracene part is suppressed by electrostatic interaction of nitrogen atoms and boron atoms in the molecule, a quenching action is eliminated, and the anthracene emits fluorescence. By this recognition mechanism, the presence of glucose can be detected as an ON-OFF fluorescence response.

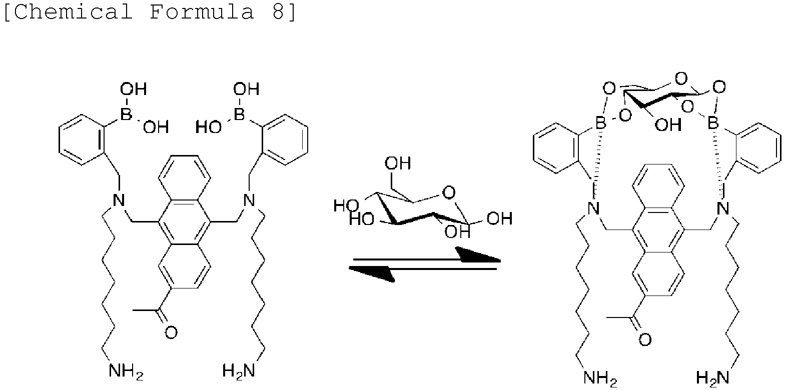

The compound of formula (III) has two amino groups at terminal ends thereof, and the amino groups react with electrophilic functional groups at terminal ends of the four-armed polymers, whereby the fluorescent dye unit can be immobilized by covalent bonding in the gel. The functional groups at the terminal ends of the compound of formula (III) are amino groups in this example, but this example is not limiting, and it would be understood by a person skilled in the art that any functional group can be used that can covalently bond with a nucleophilic functional group or an electrophilic functional group at a terminal end of the four-armed polymers. Specifically, the fluorescent dye unit can be immobilized in the gel by covalent bonding insofar as the fluorescent dye unit has any of the nucleophilic functional groups or electrophilic functional groups at a terminal end thereof.

The compound represented by formula (IV) below is cited as another preferred example of the fluorescent dye unit used in the fluorescent gel for glucose monitoring according to the present invention.

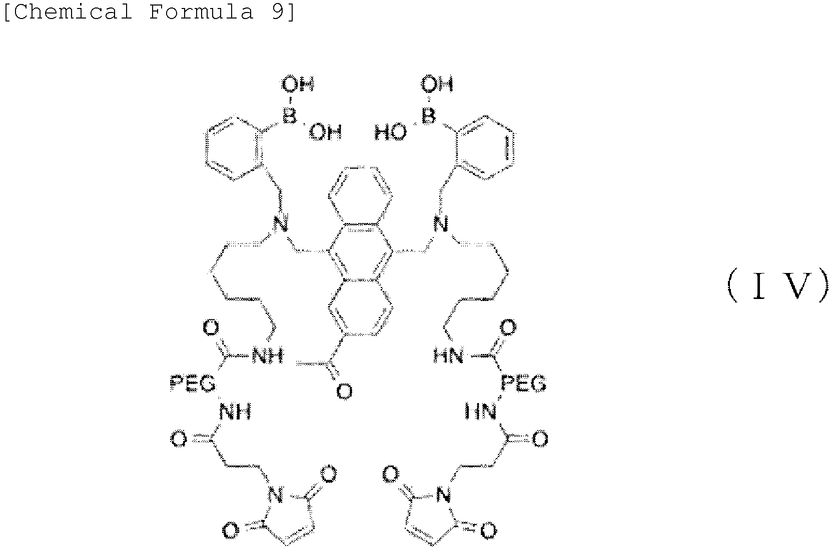

In formula (IV), "PEG" represents a polyethylene glycol chain. The polyethylene glycol chains are preferably 2-60 repeating units thereof. However, the length of the polyethylene glycol chains can be appropriately changed for use in accordance with, *inter alia,* the structure of the four-armed polymers constituting the fluorescent gel for glucose monitoring according to the present invention.

Also in the compound represented by formula (IV), the anthracene in the center part is a fluorophore, and the two arylboronic acid groups on sides thereof are quenchers, as in the compound represented by formula (III). Consequently, the mechanism for detecting the presence of glucose as an ON-OFF fluorescence response is the same as described above.

However, unlike the compound of formula (III), the compound represented by formula (IV) has two maleimide groups at terminal ends thereof. The maleimide groups can immobilize the fluorescent dye unit in the gel by covalent bonding by reacting with nucleophilic functional groups at terminal ends of the four-armed polymers.

### 3. Antioxidant component

The fluorescent gel for glucose monitoring according to the present invention may furthermore include an antioxidant in the gel. Through use of an antioxidant in the fluorescent gel, degradation of the fluorescent gel by fibrosis when the fluorescent gel is implanted in a living body can be further suppressed, and it becomes possible to further enhance durability and characteristics in continuous monitoring of glucose. Preferably, the antioxidant is catalase, superoxide dismutase (SOD), peroxiredoxin, metallothionein, glutathione peroxidase, glutathione, thioredoxin, or glucose-6-phosphate dehydrogenase (G6PD) or another enzyme. The enzyme is more preferably catalase or SOD.

The antioxidant is preferably immobilized in the gel structure by covalent bonding. A method whereby a functional group capable of covalently bonding with a nucleophilic functional group or an electrophilic functional group is introduced to the antioxidant, and the enzyme or the like is thereby immobilized in the gel by covalent bonding, likewise with respect to the fluorescent dye unit described above, can be used as the method for immobilizing the antioxidant.

### 4. Preparation of fluorescent gel for glucose monitoring

The fluorescent gel for glucose monitoring according to the present invention can be prepared by a gel preparation method which is publicly known in the technical field in question, using the fluorescent dye unit and the four-armed polymers described above, but a method may be used in which a mixed solution including a four-armed polymer (first polymer) having a nucleophilic functional group at terminal ends thereof and a fluorescent dye unit likewise having a nucleophilic functional group at terminal ends thereof, and a solution including a four-armed polymer (second polymer) having an electrophilic functional group at terminal ends thereof are mixed, for example. Crosslinking of amide bonds between the four-armed polymer components thereby occurs, a hydrogel structure having a uniform mesh structure is formed, and a gel can be manufactured in which the fluorescent dye unit is immobilized by covalent bonding.

A solution including the polymer components preferably includes an appropriate buffer solution in order to obtain an appropriate pH condition during the crosslinking reaction. A phosphate buffer solution (PBS), a citrate buffer solution, a citrate/phosphate buffer solution, an acetate buffer solution, a boric acid buffer solution, a tartaric acid buffer solution, a Tris buffer solution, a Tris-hydrochloric acid buffer solution, phosphate-buffered physiological saline, or citrate/phosphate-buffered physiological saline, a sodium chloride-sodium hydroxide buffer solution, a disodium hydrogen phosphate/sodium hydroxide buffer solution, a sodium hydrogen carbonate/sodium hydroxide buffer solution, a boric acid-potassium chloride-sodium hydroxide buffer solution, a potassium dihydrogen phosphate-sodium hydroxide buffer solution, and a potassium hydrogen phthalate-sodium hydroxide buffer solution can be cited as examples of the buffer solution used.

A step for mixing a solution of the abovementioned polymers can be performed using a two-liquid mixing syringe, for example. A temperature of the two liquids during mixing is not particularly limited, and should be such that each of the polymer components is dissolved and each of the liquids has fluidity. When the temperature is too low, compounds are not readily dissolved, or the fluidity of the solution decreases and uniform mixing is not readily obtained. When the temperature is too high, reactivity of the four-armed polymers becomes difficult to control. A temperature of 1°C to 100°C is therefore cited as the solution temperature during mixing, and a temperature of 5°C to 50°C is preferred, and 10°C to 30°C is more preferred. The two liquids may be at different temperatures, but because the two liquids are readily mixed when the temperatures thereof are the same, the temperatures of the liquids are preferably the same.

### 5. Glucose monitoring and monitoring using the fluorescent gel for glucose monitoring

The present invention furthermore relates to a method for detecting/monitoring glucose using the fluorescent gel for glucose monitoring described above, and to a sensor including the fluorescent gel.

Specifically, by bringing the fluorescent gel for glucose monitoring according to the present invention into contact with a sample as a measurement object and radiating a specific wavelength of light thereto, the presence of glucose in the sample can be detected by a fluorescence response.

The fluorescent gel for glucose monitoring according to the present invention is a biocompatible gel based on polyethylene glycol, as described above, and glucose can therefore be detected *in vivo* by implanting the fluorescent gel in a living body. Furthermore, by producing a small-sized sensor device provided with an LED or other light source, a photomultiplier tube or other detector, and a wireless data transfer system or the like as described in examples below, for example, the sensor device can be used along with the fluorescent gel as a glucose monitoring sensor which can be implanted in a living body. A fluorescence signal can be detected using a device, system, or the like which is publicly known in the technical field in question.

Furthermore, the fluorescent gel for glucose monitoring according to the present invention has characteristics whereby adhesion, etc., of proteins and the like can be suppressed and gel fibrosis can be reduced even when the fluorescent gel is implanted in a living body for a long period of time, as described above, and continuous glucose monitoring in a living body is made possible without frequent needle sampling or the like of a conventional method. The present invention is therefore suitable for use as a method or device for continuously monitoring a blood sugar level with high precision in a diabetic patient or the like.

### [Examples]

The present invention will be described in further detail below using examples, but the present invention is not limited by these examples.

### [Example 1]

### 1-1. Preparation of fluorescent gel (TAPEG-TNPEG gel)

The fluorescent gel of the present invention was prepared using the samples below.
TAPEG: Four-armed polyethylene glycol (product name: "SUNBRIGHT PTE-100PA," manufactured by YUKA SANGYO CO., LTD.) having four amino groups (nucleophilic functional groups) at the terminal ends thereof
TNPEG: Four-armed polyethylene glycol (product name: "SUNBRIGHT PTE-100PA," manufactured by YUKA SANGYO CO., LTD.) having four NHS groups (electrophilic functional groups) at the terminal ends thereof
Glucose-responsive fluorescent dye: anthracenyl arylboronic-acid derivative A (product name: "G55," manufactured by NARD INSTITUTE, LTD.)

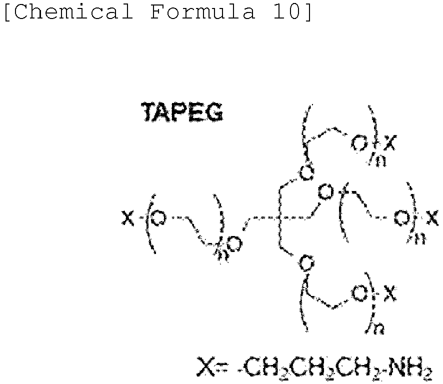

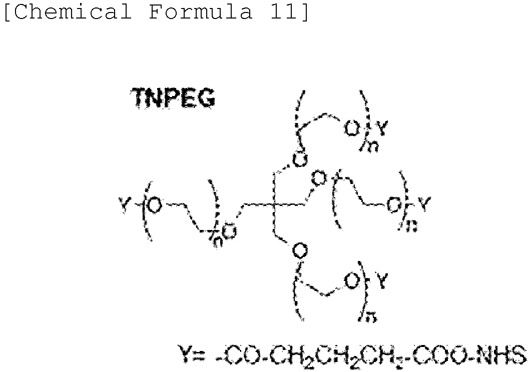

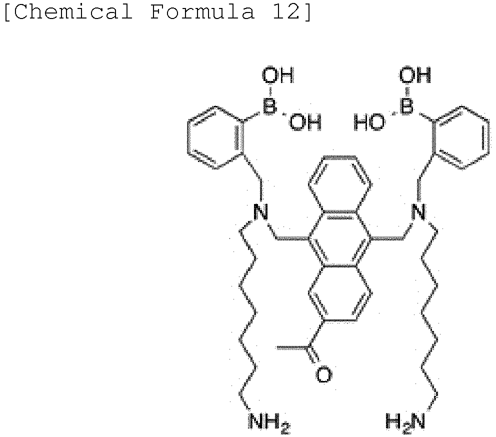

### Anthracenyl arylboronic-acid derivative A

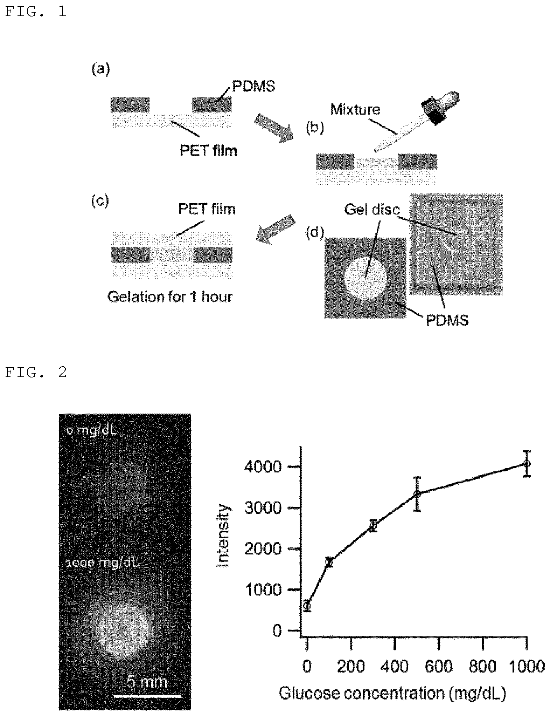

A solution was prepared in advance by dissolving 0.5 mg of the glucose-responsive fluorescent dye in 1 mL of PBS having a pH of 7.4. Two pre-gel solutions were then produced by dissolving 60 mg of TNPEG in PBS and dissolving 60 mg of TAPEG in the glucose-responsive fluorescent dye/PBS solution. The pre-gel solutions were then promptly dropped onto a PDMS mold, covered with a PET film, and left for 1 hour at 37°C (FIG. 1a-c). The PET film was peeled off, the resultant gel was placed in PBS for 1 day, and unreacted material was removed. An image of the resultant gel is shown in FIG. 1d.

Table 1 shows the results of producing gels in which a molecular weight in the above formula for TAPEG is fixed at 10000 and a molecular weight of an ether chain in the above formula for TNPEG is modified from 5000 to 20000. In Example 3, the polymer concentration in the solutions was set to 100 mg/mL.

| **Sample** | **TAPEG** | **TNPEG** | **Concentration** | **State** |
|---|---|---|---|---|
| 1 | 10000 | 10000 | 60 | Gelated (low degree of swelling) |
| 2 | 10000 | 10000 | 100 | Gelated (high degree of swelling) |
| 3 | 10000 | 5000 | 60 | Viscous liquid |
| 4 | 10000 | 20000 | 60 | Liquid |

### 1-2. Preparation of fluorescent gel (TTPEG-TMPEG gel)

The fluorescent gel of the present invention was prepared in the same manner using the samples below.
TTPEG: Four-armed polyethylene glycol (product name: "SUNBRIGHT PTE-100SH," manufactured by YUKA SANGYO CO., LTD.) having four thiol groups (nucleophilic functional groups) at the terminal ends thereof
TMPEG: Four-armed polyethylene glycol (product name: "SUNBRIGHT PTE-100MA," manufactured by YUKA SANGYO CO., LTD.) having four maleimidyl groups (electrophilic functional groups) at the terminal ends thereof
Glucose-responsive fluorescent dye: anthracenyl arylboronic-acid derivative B

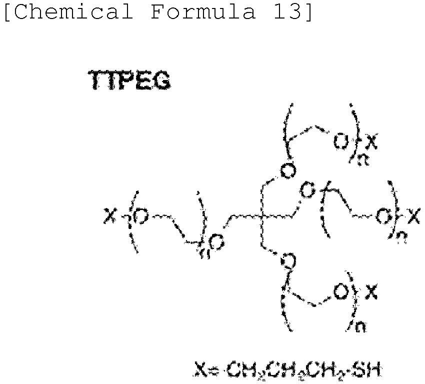

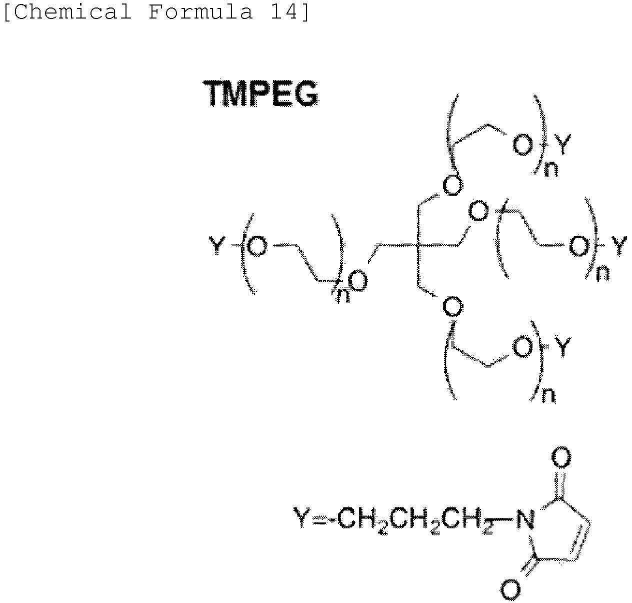

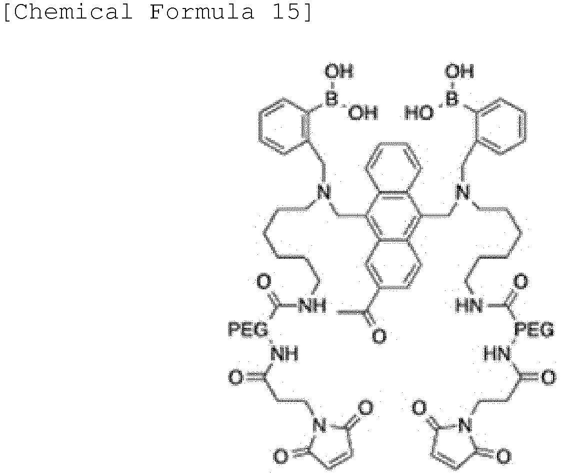

### Anthracenyl arylboronic-acid derivative B

(In the above formula, "PEG" represents a polyethylene glycol chain. The molecular weight of the polyethylene glycol chain portions was set to 2000, 3400, or 5000.)

The anthracenyl arylboronic-acid derivative B herein was synthesized by modifying terminal ends of the anthracene arylboronic-acid derivative A (product name: "G55," manufactured by NARD INSTITUTE, LTD.) used in 1-1 above. Specifically, the anthracenyl arylboronic-acid derivative A and PEG having maleimide terminal ends and a molecular weight of 2000, 3400, or 5000 (product names: "SUNBRIGHT MA-020TS," "SUNBRIGHT MA-034TS," and "SUNBRIGHT MA-050TS") were condensated in the presence of DMT-MM (condensing agent) in a mixed solvent of acetonitrile and water, and the terminal ends were modified to PEG-maleimidyl groups.

### [Example 2]

### 2. Preparation of enzyme-immobilized fluorescent gel (TAPEG-TNPEG)

A fluorescent gel was prepared in the same manner as the TAPEG-TNPEG gel of Example 1, except that a solution (TAPEG: 58 mg/mL, Fluorescent dye: 0.1 mg/mL) was used which was obtained by adding 1 mg/mL of catalase and 1 mg/mL of SOD to a mixed solution of TAPEG and the glucose-responsive fluorescent dye. Lysine groups in the enzymes in the mixed solution reacted with carboxylic acids of the gel skeleton, the enzymes were immobilized in the gel, and a fluorescent gel endowed with antioxidant ability was obtained.

A fluorescent gel was prepared in the same manner using a solution in which a composition of the mixed solution was 50 mg/mL of TAPEG, 5 mg/mL of catalase, 5 mg/mL of SOD, and 0.1 mg/mL of fluorescent dye.

It was confirmed that every fluorescent gel in which catalase and SOD were immobilized exhibited fluorescence emission as a result of addition of a 1000 mg/dL glucose solution thereto.

### [Example 3]

### 3. Evaluation (in vitro) of glucose responsiveness of fluorescent gel

Fluorescence response to glucose was evaluated using the fluorescent gels (TAPEG-TNPEG) obtained in Example 1. Glucose solutions having a concentration of 0 to 1000 mg/dL were added to each gel, and a fluorescence intensity at each concentration was measured (excitation wavelength: 405 nm, fluorescence wavelength: 490 nm). Results are shown in FIG. 2.

The fluorescence response to glucose was evaluated in the same manner also for the fluorescent gel (TTPEG-TMPEG) obtained in Example 1.

In every fluorescent gel, the fluorescence intensity was observed to increase as the glucose concentration increased. The fluorescence intensity determined uniquely with respect to glucose concentration even in repeated measurements.

### [Example 4]

### 4. Evaluation (in vivo) of glucose responsiveness of fluorescent gel

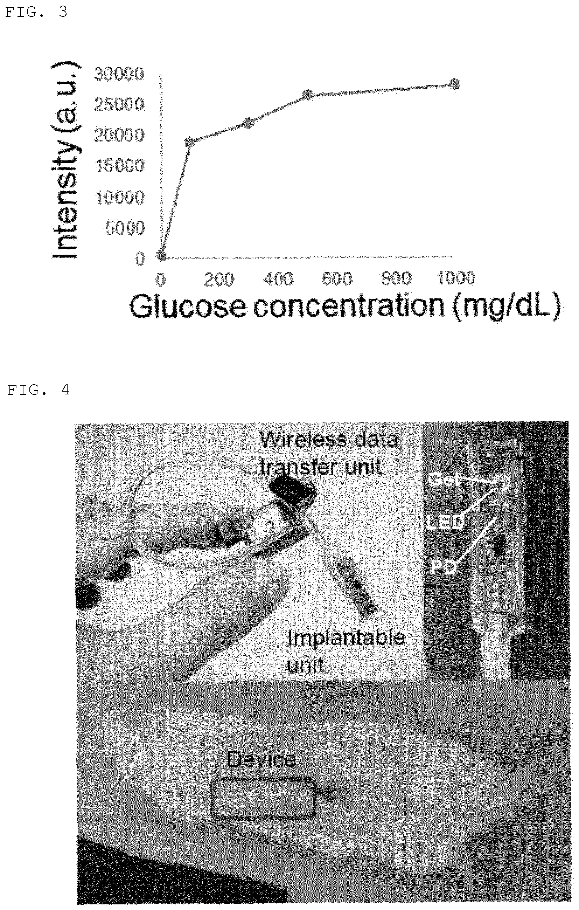

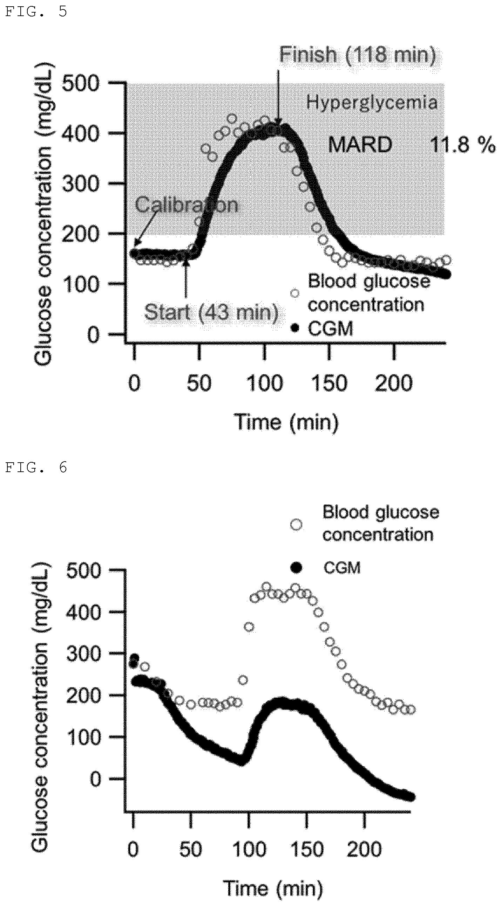

An implantable device illustrated in FIG. 4 provided with an LED light source, a Photodiode, and a wireless data transfer unit was produced, and the fluorescent gel (TAPEG-TNPEG) obtained in Example 1 was installed therein. The implantable device was implanted in a body of a mouse, a hyperglycemic state was created by administering a glucose solution in a vein, and the fluorescence response was measured. The fluorescence intensity results obtained were converted to glucose concentrations. The results thereof are shown in FIG. 5. As a comparative example, the same experiment was performed using polyacrylamide gel. The results thereof are shown in FIG. 6 in the form of a comparison with the fluorescent gel (Tetra-PEG gel) of the present invention shown in FIG. 5.

It was learned from the results in FIG. 6 that the fluorescent gel of the present invention in which Tetra-PEG is used follows an increase in glucose concentration and has higher stability than a polyacrylamide gel in glucose monitoring in a living body. In the case of polyacrylamide gel, gel degradation due to surface fibrosis a certain period of time after implantation in the body was observed, but such degradation was not seen in the fluorescent gel of the present invention in which Tetra-PEG was used.

### [Example 5]

### 5. Evaluation of durability of fluorescent gel endowed with antioxidant ability

Gel durability after implantation in the body was compared using the enzyme-immobilized fluorescent gel obtained in Example 2.

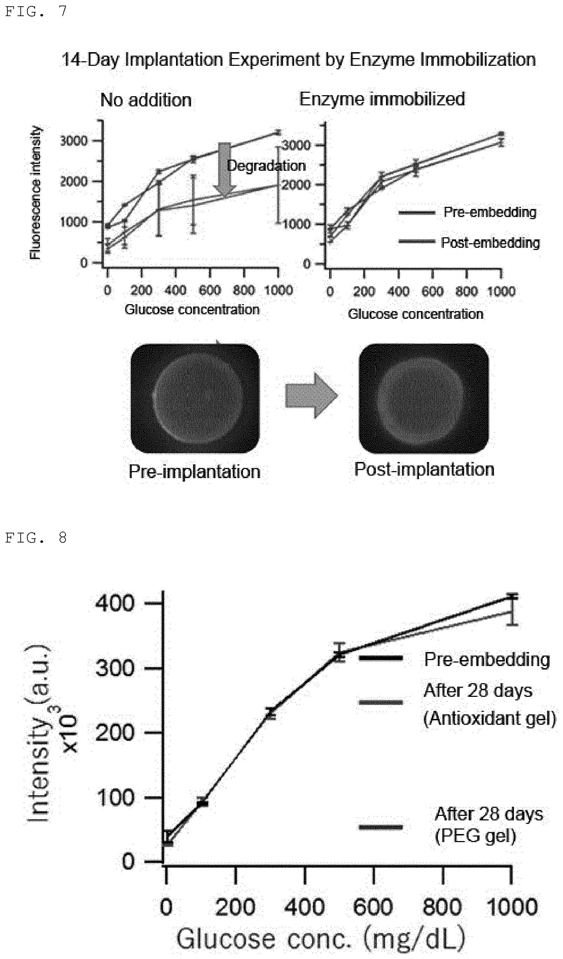

The enzyme-immobilized fluorescent gel (TAPEG-TNPEG) was implanted by the implantable device of Example 4, and the glucose response was measured at the time of implantation and at 14 days after implantation. The glucose solutions added were glucose solutions having a concentration of 0 to 1000 mg/dL. The same measurement was performed for the fluorescent gel of Example 1, in which an enzyme was not immobilized. The results are shown in FIG. 7.

As indicated in FIG. 7, in the fluorescent gel (left figure) of Example 1 in which an enzyme was not immobilized, after 14 days had elapsed since implantation, a decrease in fluorescence intensity was observed for addition of the same glucose concentrations, and a decrease in glucose responsiveness due to gel degradation by fibrosis and the like was observed. Meanwhile, in the enzyme-immobilized fluorescent gel (right figure), glucose responsiveness equivalent to that of the time of implantation was observed even after 14 days had elapsed since implantation, and it was learned that more stable continuous monitoring is possible using the enzyme-immobilized fluorescent gel.

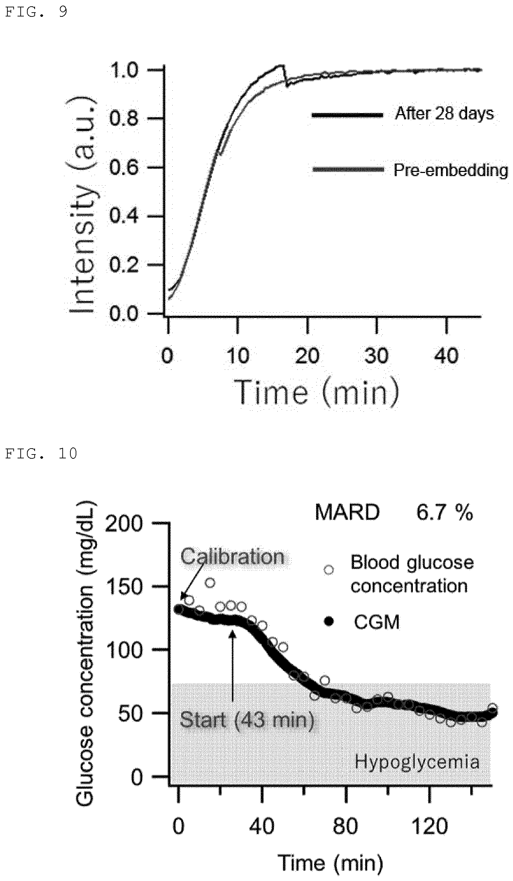

Glucose responsiveness was measured in the same manner after 28 days had elapsed since implantation, but the same fluorescence responsiveness was exhibited as prior to embedding, substantially no change in fluorescence intensity was observed, and degradation in the living body was suppressed (FIG. 8). The fluorescent gel was furthermore observed by bright field microscopy and fluorescence microscopy after 28 days had elapsed since implantation, but adhesion of fibroblasts or the like was not confirmed. Almost no difference between pre- and post-embedding was observed in glucose permeation rate before implantation and after 28 days had elapsed since embedding (FIG. 9).

### [Example 6]

### 6. Evaluation (in vivo) of glucose responsiveness in a hypoglycemic region

The same experiment as in Example 4 was performed under a condition in which insulin was administered to a diabetic mouse, and the precision of glucose monitoring in a hypoglycemic region was evaluated. Insulin at a concentration of 0.012 units/kg/min was administered 25 minutes after the start of the experiment. The results obtained are shown in FIG. 10.

As a result, it was learned that in glucose monitoring using the fluorescent gel of the present invention in which Tetra-PEG is used, high precision can be maintained even in a hypoglycemic region, in which measurement by an enzyme electrode is difficult.

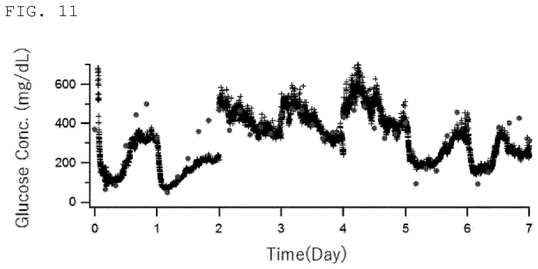

The results of performing the same glucose monitoring for one week in a diabetic mouse are shown in FIG. 11. An insulin preparation was periodically administered to produce a complex blood sugar fluctuation pattern. As a result, it was demonstrated that a glucose concentration that varies in complex fashion in a living body can be continuously measured for a long period of time.

## Claims

1. A fluorescent gel for glucose monitoring, having a three-dimensional mesh structure in which a plurality of four-armed polymers having a polyethylene glycol skeleton are crosslinked at terminal ends thereof, wherein a fluorescent dye unit for exhibiting a fluorescence response by bonding with glucose is immobilized in the gel structure by covalent bonding.

2. The fluorescent gel for glucose monitoring according to claim 1, wherein said four-armed polymers comprise a first polymer having four nucleophilic functional groups at the terminal ends thereof, and a second polymer having four electrophilic functional groups at the terminal ends thereof.

3. The fluorescent gel for glucose monitoring according to claim 2, wherein said nucleophilic functional groups are selected from the group consisting of hydroxyl groups, amino groups, and thiol groups; and said electrophilic functional groups are selected from the group consisting of carboxyl groups, N-hydroxysuccinimidyl (NHS) groups, sulfosuccinimidyl groups, maleimidyl groups, phthalimidyl groups, and nitrophenyl groups.

4. The fluorescent gel for glucose monitoring according to claim 2, wherein said first polymer has a structure represented by formula (I) 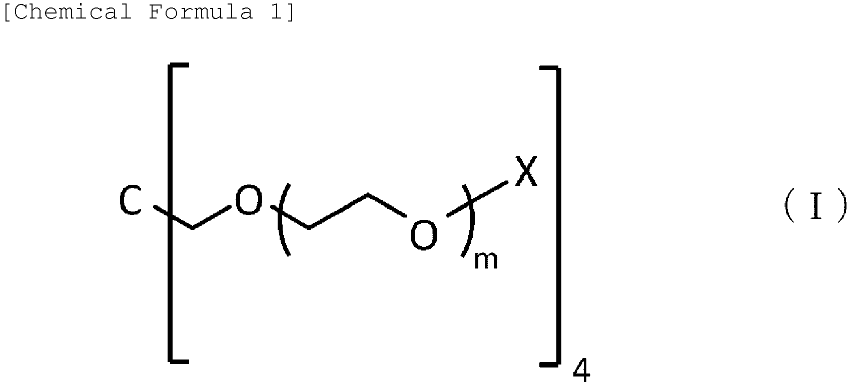 (In formula (I), each m is the same or different and is 10 to 300, each X is R¹-NH₂ or R¹-SH, and R¹ is a C₁-C₇ alkylene group); and
said second polymer has a structure represented by formula (II) 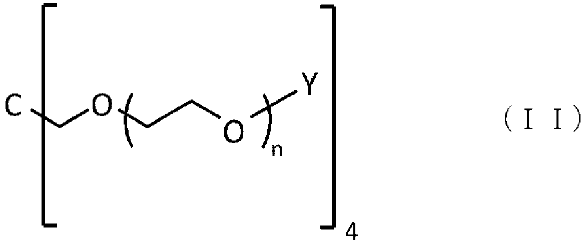 (In formula (II), each n is the same or different and is 10 to 300, and each Y is -CO-R²-COO-NHS or an R²-maleimidyl group, R² being a C₁-C₇ alkylene group.).

5. The fluorescent gel for glucose monitoring according to any one of claims 1 through 4, wherein
said fluorescent dye unit has a fluorophore and a quencher; and
said fluorophore is quenched by said quencher when glucose is not present, but by of bonding of said quencher with glucose, said fluorophore emits light and thereby exhibits a fluorescence response.

6. The fluorescent gel for glucose monitoring according to claim 5, wherein said fluorophore has anthracene and said quencher has arylboronic acid.

7. The fluorescent gel for glucose monitoring according to any one of claims 1 through 6, wherein said fluorescent dye unit has one or a plurality of amino groups at a terminal end thereof, and is immobilized in said gel structure by covalent bonding of the amino group and said four-armed polymers.

8. The fluorescent gel for glucose monitoring according to any one of claims 1 through 7, wherein said fluorescent dye unit is represented by formula (III) or formula (IV) below: 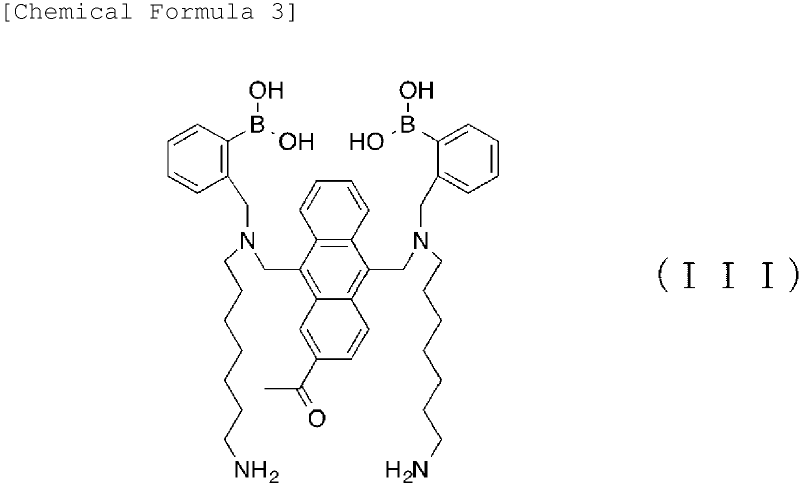 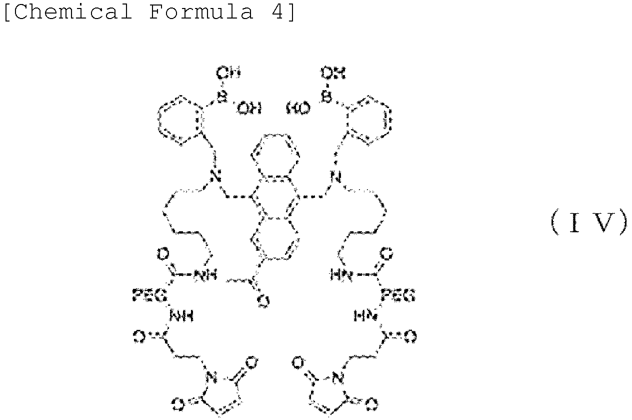 (In formula (IV), "PEG" represents a polyethylene glycol chain.).

9. The fluorescent gel for glucose monitoring according to any one of claims 1 through 8, further including an antioxidant.

10. The fluorescent gel for glucose monitoring according to any one of claims 1 through 9, wherein said antioxidant is immobilized in said gel structure by covalent bonding.

11. The fluorescent gel for glucose monitoring according to claim 9 or 10, wherein said antioxidant is an enzyme.

12. The fluorescent gel for glucose monitoring according to claim 11, wherein said enzyme is catalase, SOD, peroxiredoxin, metallothionein, glutathione peroxidase, glutathione, thioredoxin, or glucose-6-phosphate dehydrogenase.

13. An implantable glucose monitoring sensor comprising the fluorescent gel for glucose monitoring according to any one of claims 1 through 12.

14. A glucose monitoring method comprising a step for detecting, as a fluorescence response, a presence of glucose in a sample by using the fluorescent gel for glucose monitoring according to any one of claims 1 through 12.

15. A method for continuous monitoring of glucose, **characterized in that** the presence of glucose in a sample is continuously monitored by the glucose monitoring method according to claim 14.
